# EUROPEAN PATENT APPLICATION

(11) **EP 3 831 397 A1**
(43) Date of publication of application: **09.06.2021**
(21) Application number: 18927722.1
(22) Date of filing: 27.07.2018
(51) Int. Cl.: A61K 35/28, A61P 1/16, A61P 29/00, A23L 33/10

(54) **COMPOSITION COMPRISING EXOSOME AS EFFECTIVE INGREDIENT FOR PREVENTION OR TREATMENT OF ACUTE LIVER FAILURE**

(71) Applicant: Exostemtech Co., Ltd., Sangrok-gu Ansan-si, Gyeonggi-do 15588 (KR); Research & Business Foundation Sungkyunkwan University, Jangan-gu Suwon-si, Gyeonggi-do 16419 (KR)
(72) Inventor: JO, Dong-Gyu, Suwon-si, Gyeonggi-do 16363 (KR); SUL, Jae-Hoon, Uiwang-si, Gyeonggi-do 16036 (KR); KIM, Hark-Kyun, Suwon-si, Gyeonggi-do 16418 (KR); KIM, Eun-Ae, Suwon-si, Gyeonggi-do 16419 (KR); CHO, Yong-Woo, Seongnam-si, Gyeonggi-do 13530 (KR)
(74) Representative: Diehl & Partner
(86) International application number: PCT/KR2018/008557
(87) International publication number: WO 2020/022541

(57) **Abstract**

The present invention relates to a composition employing exosomes for prevention or treatment of acute liver failure. In an acute liver failure animal model, the present inventors experimentally identified that mice to which exosomes originating from adipose tissue-derived mesenchymal stem cells were injected survived at higher rates, had reduced levels of aspartate aminotransferase (AST) and alanine aminotransaminase (ALT), and were provided with excellent cell death inhibition and anti-inflammatory effects, compared with mice to which the exosomes were not injected. Thus, the composition of the present invention can be advantageously used for developing medical products and foods for preventing acute liver failure or alleviating or treating symptoms thereof.

## Description

### TECHNICAL FIELD

The present disclosure relates to a composition for preventing, improving or treating acute liver failure, which contains exosomes as an active ingredient.

### BACKGROUND ART

Acute liver failure refers to the rapid development of hepatic encephalopathy and coagulopathy within 26 weeks of the onset of any symptom in a healthy person with no history of liver disease due to the impairment of liver function.

Although the causes of acute liver failure are diverse, viral infections and medications or toxins are the common causes. The most characteristic clinical symptom is the neurological syndrome called hepatic encephalopathy. It often shows symptoms such as convulsion and delirium, and can be very risky because it can rapidly lead to coma. Hepatic encephalopathy may be accompanied by brain edema, which threatens the patient by inducing the herniation (abnormal exist of an organ from its original location) of brain stem due to rapidly increased brain pressure.

Treatment of acute liver failure can be classified into liver transplantation and treatment of various complications. Liver transplantation is the ultimate method for treating acute liver failure and is the only method capable of increasing survival rate. Nevertheless, it is not so good for acute liver failure in terms of disease progression and therapeutic result because the disease is accompanied by various severe complications including hepatic encephalopathy.

On the other hand, liver fibrosis is quite different from the acute liver failure of the present disclosure in that it is a chronic, progressive disease wherein the basic structure and function of the liver are lost as liver cells are destroyed and the normal tissue is replaced by scar tissue. It is obvious that the present disclosure is quite distinguished from the existing pharmaceutical composition, etc. for preventing liver fibrosis because it increases the survival rate of a patient and improves liver-related values by inhibiting apoptosis and alleviating inflammatory responses.

Meanwhile, mesenchymal stem cells, which are a type of adult stem cells, are multipotent cells capable of differentiating into mesenchymal tissues such as bone, cartilage, muscle, liver, etc. under specific culture conditions. They are advantageous in that they can be isolated easily and a sufficient amount can be obtained without differentiation even under normal culture conditions. Besides, they are very valuable in that they extend the survival time of transplanted cells by helping cell adherence in allogeneic cell therapy.

Recently, in the field of tissue regeneration, the therapeutic effect of paracrine secretion of substances produced by mesenchymal stem cells is being reported in addition to the cell replacement therapy using mesenchymal stem cells. Indeed, mesenchymal stem cells have been identified to secrete various substances (secretome) that induce neurogenesis, angiogenesis, anti-inflammatory response, etc. Researchers report that diseases such as cancer or heart disease can be alleviated using the secretome. For this reason, the potential of cell-free therapy using secretome has been proposed in the field of tissue therapy and regeneration. However, there are few researches on what components are included in the secretome exhibiting therapeutic effect, and further studies are necessary due to poor understanding of the mechanism and regulatory network by which it affects the recovery of damaged tissue in specific diseases.

Secretome-encapsulating exosomes are important carriers of specific components from one cell to another, and are known to be able to induce the change in gene expression in the recipient cell. Exosomes are particles with a size of 40-200 nm, which serves as extracellular vesicles and are secreted by various cells including stem cells. They have been identified in blood, body fluid, urine and cell culture. The exosomes are produced by fusion between late endosomes, which have been produced through intracellular endosomal trafficking, and the plasma membrane.

The exosomes contain not only specific proteins and mRNA transcripts expressed by the corresponding cells but also various small non-coding RNAs (microRNA, piRNA, etc.) which regulate gene expression as RNAs themselves. Accordingly, it is known that the exosomes reflect the genetic characteristics of the cells from which they are derived.

### <References of Related Art>

Korean Patent Registration No 10-1860266.
Korean Patent Registration No 10-1843634.

### DISCLOSURE

### Technical Problem

Although compositions for treating skin wound or chronic lung disease using exosomes are already known, there has been no research on treatment of acute liver failure using exosomes as in the present disclosure and more researches are necessary in this regard.

The inventors of the present disclosure have experimentally identified that an acute liver failure-induced animal model to which exosomes derived from adipose-derived mesenchymal stem cells were administered survived at higher rates, had improved levels of aspartate aminotransferase (AST) and alanine aminotransaminase (ALT) and exhibited superior apoptosis-inhibiting and anti-inflammatory effects, compared with an acute liver failure-induced animal model to which the exosomes were not administered, and have completed the present disclosure based thereon.

Therefore, the present disclosure is directed to providing a pharmaceutical composition for preventing or treating acute liver failure, which contains exosomes as an active ingredient.

The present disclosure is also directed to providing a food composition for preventing or improving acute liver failure, which contains exosomes.

The present disclosure is also directed to providing a method of treating a subject having or at risk for having acute liver failure, or a method of improving acute liver failure of the subject, which includes administering a composition comprising exosomes as an active ingredient.

The present disclosure is also directed to providing a use of exosomes in medicines or foods for treating, preventing or improving acute liver failure.

However, the technical problems to be solved by the present disclosure are not limited to those described above, and other problems not mentioned above will clearly understood by those skilled in the art from the following description.

### Technical Solution

The present disclosure provides a pharmaceutical composition for preventing or treating acute liver failure, which contains exosomes as an active ingredient.

In an exemplary embodiment of the present disclosure, the exosomes may be derived from adipose-derived mesenchymal stem cells.

In another exemplary embodiment of the present disclosure, the composition may inhibit the expression of BAX (Bcl-2-associated X protein) or may inhibit the activity of caspase-3.

In another exemplary embodiment of the present disclosure, the composition may inhibit the inactivation of PARP (poly (ADP-ribose) polymerase).

In another exemplary embodiment of the present disclosure, the composition may alleviate inflammatory responses.

In another exemplary embodiment of the present disclosure, the inflammatory responses may be inflammatory responses caused by immune cell infiltration.

In addition, the present disclosure provides a food composition for preventing or improving acute liver failure, which contains exosomes.

### Advantageous Effects

The present disclosure provides a composition for preventing, improving or treating acute liver failure using exosomes. It has been identified in an acute liver failure animal model that an acute liver failure-induced animal model to which exosomes derived from adipose-derived mesenchymal stem cells were administered survived at higher rates, had improved levels of aspartate aminotransferase (AST) and alanine aminotransaminase (ALT) and exhibited superior apoptosis-inhibiting and anti-inflammatory effects, compared with an animal model to which the exosomes were not administered. Thus, the composition of the present disclosure can be usefully used as a pharmaceutical composition, a food composition, etc. for preventing, alleviating or treating acute liver failure.

### DESCRIPTION OF DRAWINGS

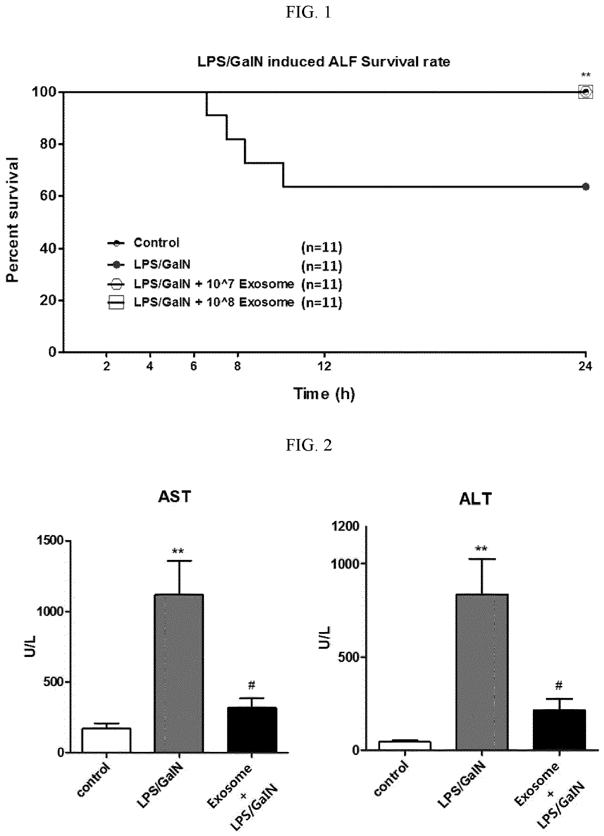

FIG. 1 shows a result of comparing the survival rate of mice to which only LPS (lipopolysaccharide)/GalN (D-(+)-galactosamine) was administered and mice to which exosomes derived from adipose-derived mesenchymal stem cells were administered together with LPS/GalN.
FIG. 2 shows a result of comparing the levels of aspartate aminotransferase (AST) and alanine aminotransaminase (ALT) in the serum of mice to which only LPS/GalN was administered and mice to which exosomes derived from adipose-derived mesenchymal stem cells were administered together with LPS/GalN.

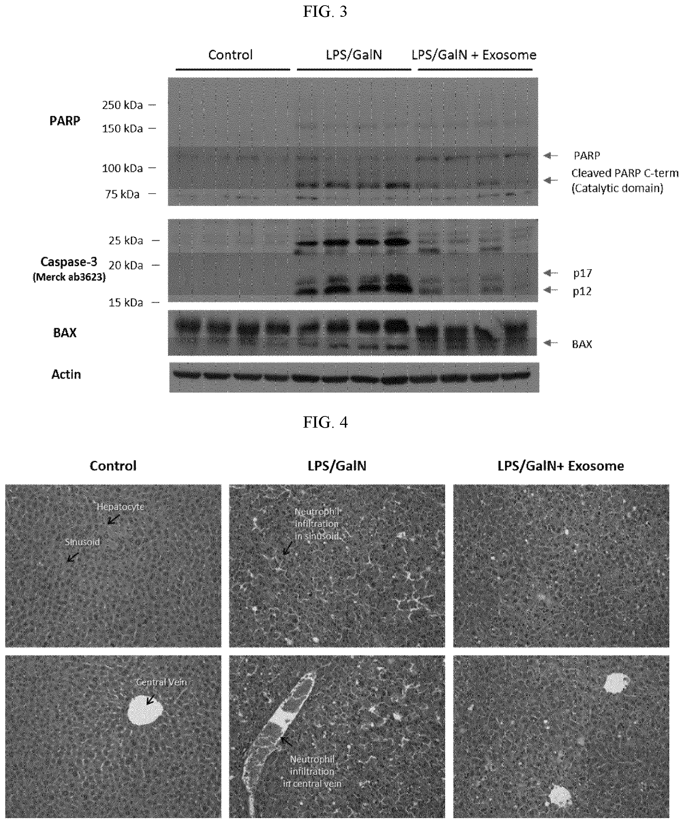

FIG. 3 shows a result of analyzing the expression of protein factors associated with apoptosis in the liver tissue of mice to which only LPS/GalN was administered and mice to which exosomes derived from adipose-derived mesenchymal stem cells were administered together with LPS/GalN.
FIG. 4 shows a result of observing the difference in inflammatory response due to immune cell infiltration in mice to which only LPS/GalN was administered and mice to which exosomes derived from adipose-derived mesenchymal stem cells were administered together with LPS/GalN by staining with hematoxylin and eosin (H&E).

### BEST MODE

The inventors of the present disclosure have experimentally identified that an acute liver failure-induced animal model to which exosomes derived from adipose-derived mesenchymal stem cells were administered survived at higher rates, had improved levels of aspartate aminotransferase (AST) and alanine aminotransaminase (ALT) and exhibited superior apoptosis-inhibiting and anti-inflammatory effects, compared with an acute liver failure-induced animal model to which the exosomes were not administered, and have completed the present disclosure based thereon.

In an example of the present disclosure, it was identified that acute liver failure-induced mice to which exosomes derived from adipose-derived mesenchymal stem cells were administered show significantly increased survival rate as compared to mice to which the exosomes were not administered (see Example 3).

In another example of the present disclosure, it was identified that acute liver failure-induced mice to which exosomes derived from adipose-derived mesenchymal stem cells were administered show improved levels of AST and ALT as compared to mice to which the exosomes were not administered (see Example 4).

In another example of the present disclosure, it was identified that acute liver failure-induced mice to which exosomes derived from adipose-derived mesenchymal stem cells were administered show inhibited expression of the apoptosis-related factor BAX (Bcl-2-associated X protein), inhibited activity of caspase-3 and decreased C-terminals of PARP (poly (ADP-ribose) polymerase) as compared to mice to which the exosomes were not administered, suggesting that apoptosis is inhibited (see Example 5).

In another example of the present disclosure, it was identified through hematoxylin & eosin staining that acute liver failure-induced mice to which exosomes derived from adipose-derived mesenchymal stem cells were administered show decreased inflammatory response as compared to mice to which the exosomes were not administered (see Example 6).

From the results of the examples of the present disclosure described above, it was identified that the exosomes derived from adipose-derived mesenchymal stem cells according to the present disclosure increases survival rate, improves the levels of aspartate aminotransferase (AST) and alanine aminotransaminase (ALT) and has apoptosis-inhibiting and anti-inflammatory effects in acute liver failure-induced mice. Thus, the exosomes can be used for preventing, improving or treating acute liver failure.

Accordingly, the present disclosure provides a pharmaceutical composition for preventing or treating acute liver failure, which contains exosomes as an active ingredient.

The term "prevention" used in the present disclosure refers to any action of inhibiting acute liver failure or delaying the onset thereof by administering the pharmaceutical composition according to the present disclosure.

The term "treatment" used in the present disclosure refers to any action of improving or favorably changing the symptoms of acute liver failure by administering the pharmaceutical composition according to the present disclosure.

The term "exosome" used in the present disclosure refers to a small vesicle of a membrane structure secreted by various cells, which is released to the extracellular environment as the multivesicular body is fused with the plasma membrane. The exosome serves to transmit the information of a genetic factor from one cell to another by encapsulating the genetic factor. It has a diameter of approximately 30-200 nm, although not being limited thereto.

The term "acute liver failure" used in the present disclosure refers to the rapid development of hepatic encephalopathy and coagulopathy within 26 weeks of the onset of any symptom in a healthy person with no history of liver disease due to the impairment of liver function. The onset of hepatic encephalopathy within 7 days after the onset of jaundice is sub-divided as hyperacute liver failure, onset within 7 days to 3 weeks as acute liver failure, and onset within 3-26 weeks as subacute liver failure. Although the causes of acute liver failure are diverse, viral infections and medications or toxins are the common causes. The most characteristic clinical symptom is the neurological syndrome called hepatic encephalopathy. It often shows symptoms such as convulsion and delirium, and can be very risky because it can rapidly lead to coma. Treatment of acute liver failure can be classified into liver transplantation and treatment of various complications. Liver transplantation is the ultimate method for treating acute liver failure and is the only method capable of increasing survival rate. The treatment of various complications should be conducted in the intensive care unit of a hospital where the liver transplantation facility is equipped after through diagnosis and examination. For acute liver failure, disease progression and therapeutic result are very poor because the disease is accompanied by various severe complications including hepatic encephalopathy.

In the present disclosure, the exosomes may be derived from adipose-derived mesenchymal stem cells, although not being limited thereto.

The term "stem cell" used in the present disclosure refers to an undifferentiated cell which is capable of self-renewal and has the capacity to differentiate into two or more different types of cells. The stem cell may be an autologous or allogeneic stem cell and may be derived from any animal including human and non-human mammals. The stem cell may be one derived from an adult or an embryo, without limitation.

The term "mesenchymal stem cell (MSC)" used in the present disclosure refers to a multipotent stem cell that can differentiate into various mesodermal cells including bone, cartilage, fat and muscle cells and ectodermal cells such as neurons.

It is known that the MSCs derived from various sources such as fat, bone marrow, umbilical cord blood, etc. have different therapeutic potentials (Jin et al., Comparative Analysis of Human Mesenchymal Stem Cells from Bone Marrow, Adipose Tissue, and Umbilical Cord Blood as Sources of Cell Therapy, Int J Mol Sci. 2013 Sep; 14(9): 17986-18001).

The exosome used as an active ingredient of the present disclosure serves as a signal transducer which transmits the information of a genetic factor from one cell to another by encapsulating the genetic factor, and exhibits different characteristics depending on the state of the cell from which the exosome is isolated.

For example, it has been found out that different bioactive factors are encapsulated in exosomes isolated from adipose-derived stem cells (human adipose-derived stem cells; Stem-EXO), exosomes isolated from epidermal keratinocytes (human epidermal keratinocytes; K-EXO) and exosomes isolated from foreskin fibroblasts (human foreskin fibroblasts; F-EXO) (WO2016-072821 A1).

In addition, it has been found out that the exosomes isolated from adipose-derived stem cells, bone marrow-derived stem cells and umbilical cord blood-derived stem cells have different characteristics depending on the sources of the stem cells.

The exosomes used in the present disclosure may be derived from adipose-derived mesenchymal stem cells. Specifically, they may be derived from proliferating adipose-derived mesenchymal stem cells. The exosomes derived from proliferating stem cell may be isolated in a process of culturing stem cells.

Although the adipose tissue-derived mesenchymal stem cells are similar to bone marrow- or umbilical cord blood-derived mesenchymal stem cell in cell morphology and immunological phenotype, they have different therapeutic potential and are advantageous over umbilical cord blood-derived mesenchymal stem cells in that yield is higher due to higher cell population frequency.

Since adipose tissue is isolated from autologous fat, there is no risk of immune rejection, etc. In addition, there is no ethical issue and it is thought that the isolation or suction of adipose tissue for isolation of mesenchymal stem cells will cause less pain and burden as compared to the bone marrow puncture for obtaining bone marrow-derived mesenchymal stem cells.

In addition, adipose-derived stem cells for isolation of exosomes can be obtained easily from abundant adipose tissue, whereas the amount of bone marrow and blood that can be obtained from a patient is very limited.

The adipose tissue may be obtained from the adipose tissue of human, rat, mouse, dog, cow, etc., although not being limited thereto. Specifically, it can be obtained from the adipose tissue of human, more specifically from the autologous adipose tissue of the patient.

In the present disclosure, the composition may inhibit the expression of BAX (Bcl-2-associated X protein) or may inhibit the activity of caspase-3.

The term "BAX (Bcl-2-associated X protein)" used in the present disclosure, also known as bcl-2-like protein 4, refers to one of Bcl-2 family proteins. The BAX promotes the apoptosis of cells. It is present mainly in a state bound to the mitochondrial outer membrane, with its four C-terminal residues protruding toward the intermembrane space between the mitochondrial inner and outer membranes. This protein is known to interact with, and increase the opening of the mitochondrial voltage-dependent anion channel (VDAC), which leads to the loss in membrane potential and the release of cytochrome c.

The term "apoptosis" used in the present disclosure refers to a form of cell death controlled by genes and is distinguished from necrosis which refers to necrotic or pathological cell death. The apoptosis is responsible for body shaping during development and renewal of normal cells or removal of abnormal cells in adults. It is distinguished from a similar process called programmed cell death (PCD) in that it occurs also in cancer cells, viral infections, drugs, radiations, etc.

The term "caspase" used in the present disclosure refers to a protease enzyme which cleaves proteins in the nucleus and the cytoplasm in the final stage of apoptosis, thereby leading to cell death.

It contains cysteine in the active site of the enzyme, which specifically recognizes aspartic acid in an amino acid sequence of a target protein and cleaves the next amino acid residue. Caspase-3 is a 32-kDa cysteine protease. It exists in the brain of mammals as an inactive zymogen which plays an important role during morphogenetic cell death as an effector caspase, and is activated as it is cleaved into 17-kDa and 12-kDa subunits.

In the present disclosure, the composition may alleviate inflammatory responses.

The term "inflammatory response" used in the present disclosure refers to the response at the wounded area, which causes heat, redness, swelling and pain. The inflammatory response is initiated by histamine released by mast cells from wound site and cytokines, etc. secreted by macrophages which are activated by engulfing bacteria.

The term "immune cell" used in the present disclosure refers to a cell participating in the innate immunity and the adaptive immunity. The immune cell is a cell which recognizes various non-self substances and defends our body from pathogens, viruses, parasites, etc. The innate immunity involves skin barrier, inflammatory responses, complement system, cellular barrier, natural killer cells, etc. and the adaptive immunity involves killer T cells, helper T cells, B lymphocytes, etc.

The pharmaceutical composition according to the present disclosure contains exosomes as an active ingredient and may contain a pharmaceutically acceptable carrier. The pharmaceutically acceptable carrier may be one commonly used for preparation and may include saline, sterile water, Ringer's solution, buffered saline, cyclodextrin, dextrose solution, maltodextrin solution, glycerol, ethanol, liposome, etc., although not being limited thereto. If necessary, it may further contain other commonly used additives such as an antioxidant, a buffer, etc. In addition, an injectable formulation such as an aqueous solution, a suspension, an emulsion, etc., a pill, a capsule, a granule or a tablet may be prepared by further adding a diluent, a dispersant, a surfactant, a binder, a lubricant, etc. Suitable pharmaceutically acceptable carriers and preparations are described in Remington's literature. The pharmaceutical composition of the present disclosure is not particularly limited in formulation and can be prepared into an injection, an inhalant, a formulation for external application to skin, a formulation for oral ingestion, etc.

The pharmaceutical composition of the present disclosure may be administered orally or parenterally (e.g., intravenously, subcutaneously, dermally, intranasally or intratracheally) depending on purposes, and the administration dosage may be selected adequately by those skilled in the art although it may vary depending on the condition and body weight of a patient, the severity of a disease, drug type, administration route and administration time.

The composition according to the present disclosure may be administered with a pharmaceutically effective amount. In the present disclosure, the "pharmaceutically effective amount" refers to an amount sufficient to treat a disease at a reasonable benefit/risk ratio applicable to medical treatment. An effective dosage level may be determined depending on a variety of factors including the type and severity of a disease, drug activity, drug sensitivity, administration time, administration route, excretion rate, treatment period and co-administered drugs, and other factors well known in the medical field. The composition according to the present disclosure may be administered either alone or in combination with other therapeutic agents. The co-administration of the composition according to the present disclosure with existing therapeutic agents may be carried out sequentially or simultaneously. Single or multiple dosages are possible. It is important to administer the composition with the minimum amount to achieve the greatest therapeutic effect without side effects, considering all the factors described above, and it may be easily determined by those skilled in the art.

Specifically, the effective amount of the composition according to the present disclosure may vary depending on the age, sex and body weight of a patient. However, the scope of the present disclosure is not limited by the administration dosage by any means because it can be increased or decreased depending on administration route, severity of the disease, sex, body weight, age, etc. of the patient, and so forth

In another aspect, the present disclosure provides a food composition for preventing or improving acute liver failure, which contains exosomes.

The food composition includes a functional health food composition.

The term "improvement" used in the present disclosure refers to any action of that at least lowers the parameters related with the condition being treated, e.g., the severity of symptoms.

In the food composition of the present disclosure, the active ingredient may be adequately added to food either alone or together with other foods or food ingredients, according to common methods. The content of the active ingredient may be determined adequately depending on the purpose of use (prevention or improvement). In general, when preparing foods or drinks, the composition of the present disclosure is added in an amount of 15 wt% or less, specifically 10 wt% or less, based on the raw material. However, in case of long-term use for the purpose of health care and hygiene, the amount may be smaller than those described above.

The food composition of the present disclosure may contain, in addition to the active ingredient of the designated content as an essential ingredient, other ingredients without special limitation. It may further contain various flavorants, natural carbohydrates, etc. as in common drinks. Examples of the natural carbohydrate are monosaccharides such as glucose, fructose, etc., disaccharides such as maltose, sucrose, etc., common sugars and polysaccharides such as dextrin, cyclodextrin, etc. and sugar alcohols such as xylitol, sorbitol, erythritol, etc. In addition, natural flavorants (thaumatin, stevia extract (e.g., rebaudioside A, glycyrrhizin, etc.)) or synthetic flavorants (saccharine, aspartame, etc.) may be used as flavorants. The content of the natural carbohydrate may be adequately selected by those skilled in the art.

In addition, the food composition of the present disclosure may contain various nutrients, vitamins, minerals (electrolytes), flavorants such as synthetic flavorants, natural flavorants, etc., colorants, extenders (cheese, chocolate, etc.), pectic acid and its salts, alginic acid and its salts, organic acids, protective colloidal thickeners, pH control agents, stabilizers, antiseptics, glycerin, alcohols, carbonating agents used in carbonated drinks, etc. These ingredients may be used either indecently or in combination. The content of these additives may also be selected adequately by those skilled in the art.

Hereinafter, specific examples are provided to help the understanding of the present disclosure. However, the following examples are provided only for easier understanding of the present disclosure and the content of the present disclosure is not limited by the examples.

### [Examples]

### Example 1. Isolation of exosomes from adipose-derived mesenchymal stem cells and analysis of characteristics

Exosomes were isolated from human adipose-derived stem cells during a procedure of culturing human adipose-derived stem cells.

Specifically, human adipose-derived stem cells were cultured in a DMEM (Dulbecco's modified Eagle's medium, high glucose) medium containing 10% fetal bovine serum (FBS) and 1% penicillin/streptomycin. 24 hours before the isolation of exosomes, the medium was replaced with a serum-free, antibiotic-free, phenol red-free medium. After culturing for 24 hours, a supernatant was recovered from the cell culture. After the supernatant was recovered, a normal culture medium was added and the stem cells were cultured again. This procedure was repeated until passage 7.

Cell debris and wastes were removed from the recovered cell culture supernatant by centrifuging at 2,000 × g and 4 °C for 10 minutes and filtering through a bottle top filter (pore size; 0.22 µm). After the primary purification, tangential flow filtration (TFF) was conducted for concentration and further purification of exosomes. For this, a filter with a molecular weight cut-off (MWCO) of 300 kDa or 500 kDa was used. During the filtration step, the exosomes were washed 1-3 times with phosphate-buffered saline (PBS).

The concentration of the isolated exosomes and the quantity of proteins were investigated by nanoparticle tracking analysis (NTA) and protein assay. For evaluation of concentration of the exosomes, the motion of each exosome was tracked from frame to frame such that the number of nanoparticles identified on the screen was 20 or larger. The concentration of the isolated exosomes was 1.78×10¹⁰ particle/mL on average, and the average size was around 211.6 nm. In addition, the average protein concentration was identified as 185.8 µg/mL by BCA protein assay.

### Example 2. Establishment of acute liver failure-induced animal model

For establishment of a fulminant liver disease animal model, 3-week-old ICR male mice were habituated for a week. After fasting for 18 hours on the previous day of 4 weeks of age, 20 µg/kg LPS (lipopolysaccharide) and 800 mg/kg GalN (D-(+)-galactosamine) were administered intraperitoneally. For preventive purpose, 10⁷ or 10⁸ exosomes were administered by intravenous injection every day from two days before the administration of LPS/GaIN, for a total of two times. For therapeutic purpose, 10⁷ or 10⁸ exosomes were administered 3 hours after the administration of LPS/GalN.

### Example 3. Confirmation of increased mouse survival rate due to administration of exosomes to acute liver failure-induced animal model

After the administration of LPS/GaIN, mouse survival rate was measured for 24 hours. The number of mice which died after the administration of LPS/GaIN was counted with time. The survival rate was plotted on a graph using the GraphPad Prism program and was analyzed statistically with the Kaplan-Meier estimator and the log-rank test. As a result, out of the 11 mice administered with LPS/GaIN only, four mice (36.36%) died, at 6 hours and 35 minutes, 7 hours and 30 minutes, 8 hours and 20 minutes, and 10 hours and 6 minutes after the administration of LPS/GaIN, respectively. In contrast, all of the 11 mice administered with LPS/GaIN and 10⁷ or 10⁸ exosomes survived after the administration of LPS/GaIN (100%). As a result of analyzing the statistical difference of the survival rate with the Kaplan-Meier estimator and the log-rank test, the group administered only with LPS/GaIN showed significantly decreased survival rate; however the groups administered with 10⁷ or 10⁸ exosomes before or after the administration of LPS/GaIN showed significant increase in survival rate.

### Example 4. Confirmation of improved liver-related values due to administration of exosomes to acute liver failure-induced animal model

For measurement of liver-related values, after administering LPS/GaIN alone or co-administering LPS/GaIN and 10⁸ exosomes, blood was taken from the hepatic portal vein or the left ventricle 5 hours later. After incubation at room temperature for 10 minutes, the blood was centrifuged at 1500 g for 10 minutes at 4 °C, and only the serum was collected from the supernatant. The collected serum was subjected to AST and ALT level analysis. As a result, the AST and ALT levels were significantly increased in the mice administered only with LPS/GaIN as compared to a control group, but were significantly decreased in the mice administered with not only LPS/GaIN but also 10⁸ exosomes.

### Example 5. Confirmation of inhibition of apoptosis due to administration of exosomes to acute liver failure-induced animal model

For confirmation of the inhibition of apoptosis in the liver tissue of mouse to which the exosomes isolated from the adipose-derived mesenchymal stem cells were administered, liver tissue was taken 5 hours after administration of LPS/GaIN alone or co-administration of LPS/GaIN and 10⁸ exosomes. After washing with PBS and removing the gallbladder, the caudate lobe was isolated from the liver tissue and was used as a sample tissue for western blot. The isolated caudate lobe was added to a lysis buffer containing a protease inhibitor and a phosphatase inhibitor and homogenized with a homogenizer. After incubation on ice for 30 minutes and centrifugation at 13,000 rpm for 10 minutes at 4 °C, only the supernatant was collected. The concentration of proteins was quantitated by BCA assay. After mixing the sample with a 4x sample buffer containing 2-mercaptoethanol, the mixture was boiled at 95 °C for 10 minutes. The active caspase-3, PARP and BAX proteins were analyzed quantitatively and qualitatively through immunoblotting. As a result, the level of the active caspase-3, BAX and cleaved PARP proteins, which are apoptotic markers, was increased in the liver tissue of the mouse treated only with LPS/GaIN, but was decreased in the mice administered with not only LPS/GaIN but also 10⁸ exosomes.

### Example 6. Confirmation of anti-inflammatory effect due to administration of exosomes to acute liver failure-induced animal model

For confirmation of the infiltration of inflammatory cells into the liver sinusoid and the central vein, mouse liver tissue was taken 5 hours after the administration of LPS/GaIN alone or co-administration of LPS/GaIN and 10⁸ exosomes. After washing with PBS and removing the gallbladder, the left lobe was isolated from the liver tissue and was used as a sample tissue for hematoxylin and eosin staining. The liver tissue was made into paraffin sections with a thickness of 5 µm and stained with hematoxylin and eosin. The immune cells that infiltrated into the sinusoid and the central vein were observed with a 40x optical microscope. As a result, whereas the infiltration of immune cells such as neutrophils and monocytes into the sinusoid and the central vein was observed for the mouse administered only with LPS/GaIN, the infiltration of the immune cells was decreased in the mice administered with not only LPS/GaIN but also 10⁸ exosomes.

## Claims

1. A pharmaceutical composition for preventing or treating acute liver failure, comprising exosomes as an active ingredient.

2. The pharmaceutical composition according to claim 1, wherein the exosomes are derived from adipose-derived mesenchymal stem cells.

3. The pharmaceutical composition according to claim 1, wherein the exosomes are derived from the autologous adipose-derived mesenchymal stem cells of a patient who will be administered with the composition.

4. The pharmaceutical composition according to claim 1, wherein the exosomes are isolated from a culture of adipose-derived mesenchymal stem cells.

5. The pharmaceutical composition according to claim 1, wherein the composition inhibits the expression of BAX (Bcl-2-associated X protein) or inhibits the activity of caspase-3.

6. The pharmaceutical composition according to claim 1, wherein the composition alleviates inflammatory responses.

7. The pharmaceutical composition according to claim 6, wherein the inflammatory responses are inflammatory responses by immune cells.

8. A food composition for preventing or improving acute liver failure, comprising exosomes.

9. A method of treating a subject having acute liver failure, comprising administering a composition comprising exosomes as an active ingredient.
